# EUROPEAN PATENT SPECIFICATION

(11) **EP 2 877 143 B1**
(45) Date of publication and mention of the grant of the patent: **05.09.2018**
(21) Application number: 13739741.0
(22) Date of filing: 24.07.2013
(51) Int. Cl.: A61K 8/34, A61K 8/41, A61K 8/04, A61Q 5/12

(54) **PROCESS**
VERFAHREN
PROCÉDÉ

(30) Priority: 27.07.2012 EP 12178168
(43) Date of publication of application: 03.06.2015
(73) Proprietor: Unilever PLC, London, Greater London EC4Y 0DY (GB); Unilever N.V., 3013 AL Rotterdam (NL)
(72) Inventor: CASUGBO, Christia, Bebington Wirral Merseyside CH63 3JW (GB); FLANAGAN, Mark, Bebington Wirral Merseyside CH63 3JW (GB); HOUGH, John, Alan, Bebington Wirral Merseyside CH63 3JW (GB); NAUGHTON, John, Michael, Bebington Wirral Merseyside CH63 3JW (GB); SERRIDGE, David, Bromborough Wirral Merseyside CH62 2FD (GB)
(74) Representative: Chisem, Janet
(86) International application number: PCT/EP2013/065647
(87) International publication number: WO 2014/016353

(56) References cited:
- EP-A1- 2 460 508
- WO-A1-2008/055816
- WO-A2-2007/136708

## Description

The present invention relates to a process for making an improved conditioning composition.

EP-A1-2 460 508 (P&G) discloses a hair conditioning composition comprising a cationic surfactant, a high melting point fatty compound and an aqueous carrier. The method for manufacture involves preparing a premix comprising the cationic surfactants and fatty compounds, wherein the temperature of the premix is higher than a melting point of the fatty compound.

WO 2008/055816 (Unilever) discloses conditioning shampoo compositions comprising a gel network that comprises a quaternary ammonium compound and a C12-C22 fatty alcohol. In the examples, cetyl alcohol is added to water at 65C with high speed stirring and then followed by adding CTAC to make a uniform dispersion at 65C. This is then added to aqueous solution at room temperature with moderate stirring.

WO 2007/136708 (P&G) discloses hair care compositions comprising an aminosilicone. The method of preparation comprises heating de-ionised water to 85C and mixing in cationic surfactants and fatty compounds. Water is maintained at 85C until the components are homogenised then the mixture is cooled to around 55C and maintained at this temperature to form a gel matrix.

Despite the prior art there remains a need for improved conditioning compositions.

Accordingly, and in a first aspect, there is provided a process for making a conditioning gel phase according to claim 1.

Conditioning compositions made using the conditioning gel phase of the invention are superior conditioning products. Specifically, they are thicker, despite having lower solids levels, and they are rinsed more easily. Products which are rinsed more easily use less water and so provide for a more sustainable future. These products are considered desirable by the environmentally aware consumer.

Preferably, the process is a continuous process.

The comelt of the invention forms an isotropic phase which means the development of structure, i.e. the formation of the lamellar conditioning gel phase, can be controlled. In this process the temperature of the mixture of comelt and water is controlled by modifying the temperature of water added to the mix. Water may be added in one go or it may be staged. Typically, a first water vessel is maintained at around 40°C and is pumped into the mixing vessel while a second water vessel is maintained at a sufficient temperature to modify the temperature of the mixture of water with comelt such that it falls within the required range, i.e. from 56-65°C, preferably from 58-62°C, more preferably 60°C in the mixing vessel.

The conditioning composition ultimately made using such conditioning gel phase exhibits improved conditioning characteristics which are not observed when the conditioning gel phase is formed in the comelt.

The improvement thus resides in the balance of thermal energy at the point of mixing the water with the comelt.

If too cold then one ends up with a poorly mixed system due to the tendency for the comelt to solidify and this ultimately provides a composition of low viscosity. If the temperature of the mix vesicles form. This also gives rise to lower viscosity in the conditioning composition formed in the long run.

Preferably, the comelt comprises from 45-90% wt. comelt fatty alcohol.

Preferably, the fatty alcohol comprises from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is particularly preferable.

The level of fatty alcohol in the conditioner of the invention (not just the conditioning gel phase) will generally range from 0.01 to 10%, preferably from 0.1% to 8%, more preferably from 0.2 % to 7 %, most preferably from 0.3 % to 6 % by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Preferably, the comelt comprises from 10-40% wt. of the comelt cationic surfactant.

Suitable conditioning surfactants include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently (C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Preferably, the cationic surfactant component of the comelt comprises from 0-70% cationic component, cationic surfactants have the formula N⁺R¹R²R³R⁴ as described above, more preferably from 30-60% wt. cationic surfactant component.

Another example of a class of suitable cationic surfactants for use in the invention, either alone or together with one or more other cationic surfactants, is a combination of (i) and (ii) below:
(i) an amidoamine corresponding to the general formula (I):

   (I) R1CONH(CH2)mN(R2)R3

   in which R¹ is a hydrocarbyl chain having 10 or more carbon atoms, R² and R³ are independently selected from hydrocarbyl chains of from 1 to 10 carbon atoms, and m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than 95 mole% (293 K) of the amidoamine present.

Should an amidoamine of the type described herein be present then the corresponding acid component will not be present in the comelt. Instead it will be present in the water. Preferably, the water comprises protonating component at from 0.01 to 3% wt.

Accordingly, where the invention requires from 10-40% wt. comelt cationic surfactant, the cationic surfactant component may comprise amidoamine which is not protonated, i.e. it will not be cationic charged but will become protonated when added to the water and hence the protonating material contained therein.

Preferably, the cationic surfactant component of the comelt comprises from 0-70% cationic component, amidoamine corresponding to formula (I), more preferably from 30-60% wt. cationic surfactant component.

In conditioning compositions of the invention (not merely the conditioning gel phase), the level of cationic surfactant will generally range from 0.01 % to 10%, more preferably 0.05 % to 7.5%, most preferably 0.1 % to 5% by weight of the composition.

Preferably, the comelt is maintained at a melting point sufficient to maintain the fatty alcohol in a liquid phase. Preferably, the comelt is maintained at from 80-85C.

Preferably, the temperature of the mixture of the comelt and the water is controlled such that it is maintained from 56-65C, prefer from 58-62C, more preferably 60C during mixing.

Preferably, the contents of the mixture vessel passed through a mixer with rotor tip speed of 10-34, preferably from 21-27 and especially preferably 24 ms-1.

In a second aspect there is provided a process for manufacturing a conditioning composition by forming a conditioning gel phase obtained by the first aspect and then adding any remaining ingredients. Typical remaining ingredients include fragrances, silicones, fibre actives or other benefit agents.

Preferably, the conditioning composition is passed through a mixer with rotor tip speed of 10-34, preferably from 21-27 and especially preferably 24 ms-1 one more time after the remaining ingredients have been added. Conditioning compositions of the invention or using conditioning gel phases of the invention also deposit silicone better than conventionally made conditioning compositions.

Accordingly, the compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25°C the viscosity of the silicone itself is preferably at least 60,000 cst, most preferably at least 500,000 cst, ideally at least 1,000,000 cst. Preferably the viscosity does not exceed 10⁹ cst for ease of formulation

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of 0.15 micron are generally termed microemulsions.

Emulsified silicones for use in the conditioner compositions of the invention will typically have a size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant the invention are amino functional silicones. By "amino functional silicone" is meant quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

### EXAMPLES

| **INCI** | **Active Level** | **A** | **1** |
|---|---|---|---|
| Dimethicone/amodimethicone/ Cetrimonium Chloride | 70 | 4.29 | 4.29 |
| Behentrimonium Chloride | 70 | 2.86 | 2.86 |
| Cetearyl Alcohol | 100 | 4 | 4 |
| Preservative | 55 | 0.1 | 0.1 |
| Potassium Chloride | 100 | 0.1 | 0.1 |
| Parfum | 100 | 0.6 | 0.6 |
| Preservative | 100 | 0.04 | 0.04 |
| Aqua | 100 | To 100 | To 100 |

Formulation A is made by standard process mixing the solids with the water at around 70°C while formulation 1 is made by adding cationic surfactants to fatty alcohol and stir at 85°C.

Inject this mixture into a flowing stream of water, containing other ingredients, the temperature of the water varied to ensure this mixture has a temperature of 60°C and mix.

Cool this stream towards ambient by injection into a second water stream and mix.

The compositions have different levels of conditioning active to demonstrate the improved conditioning performance of the composition made by the claimed process.

| | **A** | **1** | **Significance** |
|---|---|---|---|
| No of Panellists | 36 | 36 | |

| **Conditioner in use** | | | |
|---|---|---|---|
| Con Thickness | 56.28 D | 77.43 A | 99.9% |
| Ease Rinsing | 72.47 A | 64.60 C | 99.9% |
| Smooth Wet | 66.09 b | 72.53 a | 90% |
| Detangling | 68.53 D | 75.34 AB | 99% |

| **Dry** | | | |
|---|---|---|---|
| Smoothness | 68.30 B | 73.70 A | 95% |

Confidential in homes panel data with approx 40 panellists with damaged hair. Assessment via line scale.

The data shows that using a better process we have a thicker product despite having lower total solids (i.e. FA and BTAC). The ingredients are being used more efficiently.

In addition, the product is both significantly more conditioning (easier to detangle + more smooth) than control.

## Claims

1. Process for making a conditioning gel phase comprising:
- forming a 'comelt' in a first vessel comprising fatty alcohol and cationic component and 0-15% wt. comelt water
- independently adding the 'comelt' and water to a mixing vessel
- mixing,
wherein the temperature of the mixture of the 'comelt' and the water is maintained at from 56-65°C, preferably from 58-62°C, more preferably 60°C when in the mixing vessel by adding water heated to a sufficient temperature to the mixture, wherein the fatty alcohol comprises from 8 to 22 carbons, wherein the cationic component comprises from 0-70% cationic surfactants have the formula N⁺R¹R²R³R⁴, more preferably from 30-60% wt. cationic surfactant component, and wherein R¹, R², R³ and R⁴ are independently (C₁ to C₃₀) alkyl or benzyl.

2. Process according to claim 1 wherein the comelt comprises from 45-90% wt. comelt fatty alcohol.

3. Process according to claim 1 or 2 wherein the comelt comprises from 10-40% cationic component.

4. Process according to any preceding claim wherein the comelt is maintained at a melting point sufficient to maintain the fatty alcohol in a liquid phase, preferably at from 80-85°C.

5. Process according to any preceding claim wherein the mixture passes through a mixer with rotor tip speed of 10-34 ms-1.

6. Process for manufacturing a conditioning composition by forming a conditioning gel phase obtained by any of claims 1-5 and then adding any remaining ingredients.

7. Process according to claim 6 comprising passing the composition through a mixer with rotor tip speed of 10-30 ms-1.

8. Process according to any preceding claim which is a continuous process.

## Patentansprüche

1. Verfahren zur Herstellung einer konditionierenden Gelphase, umfassend:
- Ausbilden einer "Co-Schmelze" in einem ersten Behälter, umfassend Fettalkohol und kationische Komponente und 0-15 Gew.-% Co-Schmelze-Wasser,
- davon unabhängiges Zufügen der "Co-Schmelze" und von Wasser zu einem Mischbehälter,
- Mischen,
wobei die Temperatur der Mischung der "Co-Schmelze" und des Wassers bei 56-65°C, vorzugsweise von 58 - 62°C, bevorzugter 60°C, aufrechterhalten wird, wenn in dem Mischbehälter, durch Zufügen von Wasser, das auf eine ausreichende Temperatur erhitzt ist, zu der Mischung, wobei der Fettalkohol von 8 bis 22 Kohlenstoffatome umfasst, wobei die kationische Komponente von 0-70% kationische Tenside, die die Formel N⁺R¹R²R³R⁴ aufweisen, bevorzugter von 30-60 Gew.-% kationische Tensid-Komponente umfaßt, und wobei R¹, R², R³ und R⁴ unabhängig voneinander (C₁- bis C₃₀)-Alkyl oder Benzyl darstellen.

2. Verfahren nach Anspruch 1, wobei die Co-Schmelze von 45-90 Gew.-% Co-Schmelze-Fettalkohol umfasst.

3. Verfahren nach Anspruch 1 oder 2, wobei die Co-Schmelze von 10-40% kationische Komponente umfasst.

4. Verfahren nach irgendeinem vorhergehenden Anspruch, wobei die Co-Schmelze bei einem Schmelzpunkt aufrechterhalten wird, der ausreicht, um den Fettalkohol in flüssiger Phase, vorzugsweise bei von 80-85°C, aufrechtzuerhalten.

5. Verfahren nach irgendeinem vorhergehenden Anspruch, wobei die Mischung einen Mischer mit einer Rotorspitzengeschwindigkeit von 10-34 ms⁻¹ durchströmt.

6. Verfahren zur Herstellung einer konditionierenden Zusammensetzung durch Ausbilden einer konditionierenden Gelphase, erhalten nach irgendeinen der Ansprüche 1-5, und darauf Zufügen aller verbleibenden Bestandteile.

7. Verfahren nach Anspruch 6, umfassend das Strömen der Zusammensetzung durch einen Mischer mit einer Rotorspitzengeschwindigkeit von 10-30 ms⁻¹.

8. Verfahren nach irgendeinem vorhergehenden Anspruch, das ein kontinuierliches Verfahren darstellt.

## Revendications

1. Procédé pour préparer une phase de gel conditionneur comprenant :
- la formation d'un "produit de co-fusion" dans une première cuve comprenant un alcool gras et un composant cationique et 0 à 15 % en poids d'eau de co-fusion
- l'addition indépendante du "mélange de co-fusion" et de l'eau dans une cuve de mélange
- le mélange,
dans lequel la température du mélange du "produit de co-fusion" et de l'eau est maintenue à une valeur de 56 à 65°C, de préférence de 58 à 62°C, mieux encore de 60°C lorsqu'il est dans la cuve de mélange par addition au mélange d'eau chauffée à une température suffisante, dans lequel l'alcool gras comprend de 8 à 22 carbones, dans lequel le composant cationique comprend de 0 à 70 % de tensioactifs cationiques de formule N⁺R¹R²R³R⁴, mieux encore de 30 à 60 % en poids de composant tensioactif cationique, et dans lequel R¹, R², R³ et R⁴ sont indépendamment des radicaux alkyle en (C₁ à C₃₀) ou benzyle.

2. Procédé selon la revendication 1, dans lequel le produit de co-fusion comprend de 45 à 90 % en poids d'alcool gras de co-fusion.

3. Procédé selon la revendication 1 ou 2, dans lequel le produit de co-fusion comprend de 10 à 40 % de composant cationique.

4. Procédé selon l'une quelconque des revendications précédentes, dans lequel le produit de co-fusion est maintenu à un point de fusion suffisant pour maintenir l'alcool gras en phase liquide, de préférence à une température de 80 à 85°C.

5. Procédé selon l'une quelconque des revendications précédentes, dans lequel le mélange passe dans un mélangeur avec une vitesse de pointe de rotor de 10 à 34 m/s.

6. Procédé pour fabriquer une composition de conditionnement par formation d'une phase de gel conditionneur obtenue par l'une quelconque des revendications 1 à 5 et ensuite addition de quelconques ingrédients restants.

7. Procédé selon la revendication 6, comprenant le passage de la composition dans un mélangeur avec une vitesse de pointe de rotor de 10 à 30 m/s.

8. Procédé selon l'une quelconque des revendications précédentes, qui est un procédé en continu.
